# EUROPÄISCHE PATENTSCHRIFT

(11) **EP 0 568 848 B1**
(45) Veröffentlichungstag und Bekanntmachung des Hinweises auf die Patenterteilung: **17.06.1998**
(21) Anmeldenummer: 93106215.2
(22) Anmeldetag: 16.04.1993
(51) Int. Cl.: C11D 3/20, C11D 1/90, C11D 1/72

(54) **Verfahren zur Herstellung fliessfähiger wässriger Perlglanzdispersionen**
Process for the preparation of free flowing aqueous dispersions with pearly lustre
Procédé pour la préparation des dispersions aqueuses à lustre nacré s'écoulant bien

(30) Priorität: 27.04.1992 DE 4213614
(43) Veröffentlichungstag der Anmeldung: 10.11.1993
(73) Patentinhaber: Clariant GmbH, 65929 Frankfurt am Main (DE)
(72) Erfinder: Schmitt, Norbert, Dipl.-Ing., D-84508 Burgkirchen (DE); Reng, Alwin, Dipl.-Ing., W-6233 Kelkheim-Fischbach (DE); Gohlke, Fritz Joachim, Dr., W-8269 Burgkirchen (DE)

(56) Entgegenhaltungen:
- DE-A- 3 411 328
- DE-A- 3 843 472
- DERWENT ACCESSION Nr. 91-012 367, Questel Telesystems (WPIL) DERWENT PUBLICATIONS LTD., London
- DERWENT ACCOSSION Nr. 84-267 232, Questel Telesystems (WPIL) DERWENT PUBLICATIONS LTD., London
- DERWENT ACCESSION Nr. 92-203 882, Questel Telesystems (WPIL) DERWENT
- PUBLICATIONS LTD., London

## Beschreibung

Die Erfindung betrifft fließfähige wäßrige Perlglanzdispersionen mit Fettsäureglykolestern als Perlglanzbildner.

Es ist häufig erwünscht, Tensidkompositionen, zum Beispiel flüssigen Wasch- und Reinigungsmitteln (das sind Bodenreinigungsmittel, Geschirrspülmittel und dergleichen) oder flüssigen Körperreinigungs- und Körperpflegemitteln sowie Haarbehandlungsmitteln (das sind Shampoos, Badezusätze, Hautbehandlungslösungen und dergleichen), ein verbessertes optisches Aussehen zu verleihen. Dazu werden den Tensidkompositionen wäßrige Perlglanzdispersionen (Perlglanzkonzentrate) in einer ausreichenden Menge einverleibt, wodurch sie ein perlglänzendes und damit ästhetisch ansprechendes Aussehen erhalten.

Die bekannten wäßrigen Perlglanzdispersionen bestehen im wesentlichen aus mindestens einer perlglanzgebenden Verbindung, mindestens einem Dispergiermittel (auch Lösemittel, Netzmittel oder Emulgator genannt) und Wasser. Im Stand der Technik werden zahlreiche perlglanzbildende Verbindungen und Verbindungsmischungen empfohlen, zum Beispiel Fettsäuren, Metallsalze von Fettsäuren, Fettsäuremono- oder Fettsäuredialkanolamide, Monoester oder Diester von Alkylenglykolen wie Ethylenglykol, Propylenglykol oder Oligomeren davon mit Fettsäuren (Fettsäureglykolester), Monoester oder Polyester von Glycerin mit höheren Fettsäuren und Ketosulfone verschiedenster Art. Noch zahlreicher sind die Verbindungen, die als Dispergiermittel beschrieben sind, zum Beispiel Alkylsulfate, Alkylethersulfate, Alkylsulfonate, Aminoxide, Betaine, Anlagerungsprodukte von Ethylenoxid und/oder Propylenoxid an Fettalkohole, Fettsäuren oder Alkylphenole, Glycerinmono- oder Glycerindiester oder Sorbitanmono- oder Sorbitandiester von Fettsäuren, wobei die Glycerinester und Sorbitanester gegebenenfalls ethoxyliert und/oder propoxyliert sind, Alkylmono- oder Alkyloligoglykoside und dergleichen.

So sind in der US-Patentschrift 4,620,976 wäßrige fließfähige Perlglanzdispersionen beschrieben, die als Perlglanzkomponente eine Kombination von einem Fettsäureglykolester und einem Fettsäurealkanolamid und als Dispergiermittel ein System von tensidischen Verbindungen enthalten, nämlich Glykolethersulfosuccinaten und Aminoxiden. Trotz ihrer relativ guten Eigenschaften lassen diese Konzentrate hinsichtlich perlglanzgebender Wirkung noch zu wünschen übrig.

In DE-A-3843472 wird ebenfalls eine fließfähige wäßrige Perlglanzdispersion beschrieben, die neben vielen möglichen Perlglanzbildnern und Dispergiermitteln 0,1 bis 5 Gew.-% von einem niedrigen oder höheren mehrwertigen Alkohol als kritische Komponente enthält.

Es werden insbesondere wäßrige Perlglanzdispersionen beschrieben, die im wesentlichen bestehen aus a) 25 Gew.-% Ethylenglykoldistearat, b) 6 bis 9 Gew.-% Cocamidopropylbetain der Formel R-CONH-(CH₂)₃-N⁺(CH₃)₂-CH₂COO⁻, c) 10 bis 13 Gew.-% C₁₂₋₁₄-Alkyl-(OCH₂CH₂)₄OH [alleine oder im Gemisch mit C₁₂₋₁₄-Alkyl-(OCH₂CH₂)₃OH] und d) Rest Wasser (abzüglich 1 bis 5 Gew.-% Alkohol und Konservierungsmittel). Zur Herstellung der Dispersionen werden zunächst die Komponenten a), b) und c) auf 75 °C und gemeinsam geschmolzen. Zu der entstandenen Schmelze wird auf 75 °C erwärmtes Wasser zugegeben, und die so erhaltene Dispersion wird unter Rühren auf 25 °C abgekühlt. Alternativ kann auch die Komponente c) in Wasser gelöst und zu der Schmelz aus a) und b) gegeben werden.

Die Aufgabe der vorliegenden Erfindung besteht demnach darin, ein gutes technisches Verfahren zur Herstellung einer fließfähigen und damit leicht handhabbaren wäßrigen Perlglanzdispersion mit ausgezeichnetem Perlglanzcharakter zur Verfügung zu stellen. Das wäßrige Perlglanzkonzentrat soll in die üblichen Tensidkompositionen ohne Schwierigkeiten inkorporiert werden können und ihnen auch schon bei Einsatz einer relativ geringen Menge an Perlglanzkonzentrat perlglänzendes Aussehen verleihen.

Die wäßrige Perlglanzdispersion besteht im wesentlichen aus
a) 15 bis 35 Gew.-%, vorzugsweise 20 bis 30 Gew.-%, von mindestens einem Fettsäureglykolester der allgemeinen Formel 1 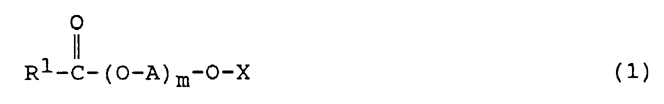 worin R¹ einen Alkylrest mit 12 bis 22 C-Atomen, A eine Gruppe der Formel -C₂H₄- oder -C₃H₆-, X ein Wasserstoffatom oder eine Gruppe der Formel 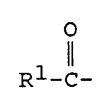 und m eine Zahl von 1 bis 10 bedeuten,
b) 5 bis 15 Gew.-%, vorzugsweise 7 bis 12 Gew.-%, von mindestens einem Betain der allgemeinen Formel 2 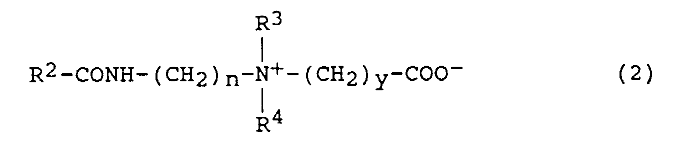 worin
   R² einen Alkylrest mit 8 bis 22 C-Atomen,
   R³ und R⁴ je einen Alkylrest mit 1 bis 3 C-Atomen,
   n 2, 3 oder 4 und y 1, 2 oder 3 bedeuten,
c) 5 bis 18 Gew.-%, vorzugsweise 7 bis 15 Gew.-%, von mindestens einem Fettalkoholalkoxylat der allgemeinen Formel 3

   R⁵-(O-B)_{z}-OH (3)

   worin
   R⁵ einen Alkylrest mit 8 bis 22 C-Atomen,
   B eine Gruppe der Formel -C₂H₄- oder -C₃H₆- und
   z eine Zahl von 1 bis 15 bedeuten, und
d) Wasser in der auf 100 Gew.-% fehlenden Menge (Gewichtsprozente bezogen auf das Gewicht der fertigen wäßrigen Dispersion).

Die Erfindung basiert auf der Feststellung, daß unter den vielen perlglanzgebenden Verbindungen gerade Fettsäureglykolester geeignet sind, wenn als Dispergiermittel eine Kombination aus zwei bestimmten tensidischen Verbindungen, nämlich einem Betain und einem Fettalkoholalkoxylat, eingesetzt wird und die fertige wäßrige Dispersion die perlglanzgebende Komponente, die beiden ausgewählten dispergierenden Verbindungen und das Wasser in einer jeweils bestimmten Menge enthält.

Bevorzugte Fettsäureglykolester sind solche gemäß Formel 1, wenn R¹ ein Alkylrest mit 14 bis 18 C-Atomen ist, A -C₂H₄- ist, X R¹-CO- ist und m eine Zahl von 1 bis 3 ist. Da es sich beim Alkylrest R¹ um Fettsäurereste handelt, kann dieser Rest auch Doppelbindungen enthalten, vorzugsweise 1 bis 3. Bevorzugte Vertreter der Komponente a) sind also Diester des Ethylenglykols, Diethylenglykols oder Triethylenglykols mit Palmitinsäure, Stearinsäure, Ölsäure, Talgfettsäure, Cocosfettsäure und dergleichen.

Bevorzugte Betaine sind solche gemäß Formel 2, wenn R² ein Alkylrest mit 10 bis 18 C-Atomen ist, R³ und R⁴ gleich und Methyl oder Ethyl sind, n 3 ist und y 1 ist. Da es sich beim Alkylrest R² ebenfalls um Fettsäurereste handelt, kann dieser Rest auch Doppelbindungen enthalten, vorzugsweise 1 bis 3. Bevorzugte Vertreter der Komponente b) sind also Lauroylaminopropyl-dimethylammoniumglycinat, Stearoylaminopropyl-dimethylammoniumglycinat, Cocosacylaminopropyl- dimethylammoniumglycinat, Talgacylaminopropyl-dimethylammoniumglycinat und dergleichen.

Bevorzugte Fettalkoholalkoxylate sind solche gemäß Formel 3, wenn R⁵ ein Alkylrest mit 10 bis 18 C-Atomen ist, B -C₂H₄- ist und z eine Zahl von 2 bis 6 ist. Auch hier kann der Alkylrest R⁵ Doppelbindungen enthalten, vorzugsweise 1 bis 3. Bevorzugte Vertreter der Komponente c) sind also die Anlagerungsprodukte von 2 bis 6 Ethylenoxideinheiten an Decylalkohol, Laurylalkohol, Stearylalkohol, Cocosalkylalkohol, Talgalkylalkohol und dergleichen.

Die erfindungsgemäße Herstellung der wäßrigen Perlglanzdispersionen erfolgt bevorzugt nach der folgenden Arbeitsweise. Die Komponenten b), c) und d), das sind die Betainverbindung, das Fettalkoholalkoxylat und das Wasser, werden in einem beheizbaren Kessel vorgelegt. Die Mischung wird unter Rühren auf 70 bis 90 °C erhitzt, um das Betain und das Fettalkoholalkoxylat in Lösung zu bringen. Getrennt von diesem Vorgang wird die Komponente a), das ist der Fettsäureglykolester, in einem zweiten Kessel homogen aufgeschmolzen durch Erhitzen auf eine Temperatur, die etwa 5 bis 20 °C oberhalb des Schmelzpunktes liegt, das ist im allgemeinen eine Temperatur von 70 bis 90 °C. Die homogen aufgeschmolzene Komponente a) wird nun bei der angegebenen Temperatur von 70 bis 90 °C unter Rühren in die Mischung aus den Komponenten b), c) und d) eingebracht. Die erhaltene Dispersion aus den Komponenten a) bis d) wird zur Temperierung bei einer Temperatur von 70 bis 90 °C, vorzugsweise 75 bis 85 °C, unter Rühren gehalten (etwa 20 bis 40 Minuten lang), worauf die Dispersion unter Weiterrühren auf eine Temperatur von 30 bis 40 °C abkühlen gelassen wird (dieses Abkühlen erfolgt also ohne Anwendung von Kälte). Während des Abkühlens kristallisiert die Komponente a) aus und bildet den gewünschten Perlglanz. Nach Erreichen einer Temperatur im genannten Bereich von 30 bis 40 °C wird die Dispersion unter Weiterrühren mit Hilfe von Kälte zügig (das heißt etwa 0,5 °C/min) abgekühlt bis auf Raumtemperatur (das sind etwa 20 bis 25 °C), womit die fertige wäßrige Perlglanzdispersion vorliegt.

Die wäßrigen Perlglanzdispersionen können neben den beschriebenen Komponenten a) bis d) noch zweckmäßige Zusätze enthalten, zum Beispiel Konservierungsmittel, Puffersubstanzen und dergleichen. So können zur Konservierung Natriumchlorid, Kaliumchlorid und/oder Magnesiumchlorid oder Ameisensäure in einer Menge von 0,1 bis 3 Gew.-% (bezogen auf die wäßrige Dispersion) zugesetzt werden. Die wäßrige Dispersion hat einen pH-Wert von 3 bis 8.

Die wäßrigen Perlglanzdispersionen sind gut fließfähig (gießfähig, pumpfähig) und damit gut handhabbar. Aufgrund ihrer niedrigen Viskosität können die Dispersionen auf automatischen Pump-, Dosier- und Mischanlagen ohne Schwierigkeiten eingesetzt werden. Die erfindungsgemäße Dispersion besitzt einen ausgeprägten Perlglanz und ist in den eingangs erwähnten Tensidkompositionen leicht einarbeitbar, wodurch diese den angestrebten Perlglanz erhalten. Die erforderliche Menge an Perlglanzdispersion liegt bei 1 bis 10 Gew.-%, vorzugsweise 2 bis 5 Gew.-%, bezogen auf das Gewicht der zu behandelnden Tensidkomposition. Das erfindungsgemäße Perlglanzkonzentrat wird also in der angegebenen Menge den Tensidkompositionen unter Rühren zugesetzt und unter Weiterrühren darin gut verteilt.

Die Erfindung wird nun an Beispielen noch näher erläutert.

Die folgenden Beispiele 1 bis 3 geben drei wäßrige Perlglanzdispersionen an (die Menge der einzelnen Komponenten sind Gewichtsprozente). Das Zusammenbringen der Komponenten, das heißt die Herstellung der wäßrigen Perlglanzdispersionen gemäß den Beispielen 1 bis 3, erfolgte nach der oben beschriebenen erfindungsgemäßen Arbeitsweise. Die erhaltenen wäßrigen Dispersionen wurden auf ihr Fließverhalten (Viskosität) bei 20 °C und auf ihren Perlglanz getestet. Beide Teste erfolgten visuell, wobei die Noten sehr gut und gut gegeben wurden. Die Testergebnisse sind am Ende eines jeden Beispiels angeführt.

### Beispiel 1

a) 30,0 % Monoethylenglykoldistearat
b) 7,0 % Cocamidopropylbetain
c) 8,0 % Laurylalkohol + 4 Ethylenoxid
d) 55,0 % Wasser
   - Fließfähigkeit:: sehr gut
   - Perlglanz:: sehr gut

### Beispiel 2

a) 20,0 % Monoethylenglykoldistearat
b) 12,0 % Cocamidopropylbetain
c) 12,0 % Laurylalkohol + 4 Ethylenoxid
d) 56,0 % Wasser
   - Fließfähigkeit:: sehr gut
   - Perlglanz:: sehr gut

### Beispiel 3

a) 25,0 % Monoethylenglykoldistearat
b) 9,0 % Cocamidopropylbetain
c) 10,0 % Laurylalkohol + 4 Ethylenoxid
d) 55,7 % Wasser
e) 0,3 % Ameisensäure als Konservierungsmittel
   - Fließfähigkeit:: sehr gut
   - Perlglanz:: sehr gut

Cocamidopropylbetain ist die Kurzbezeichnung für 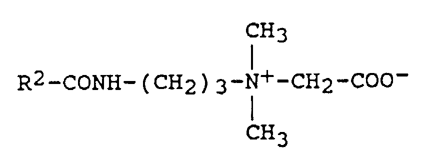 R² = Cocosalkyl

## Patentansprüche

1. Verfahren zur Herstellung von fließfähigen wäßrigen Perlglanzdispersionen bestehend aus
a) 15 bis 35 Gew.-% von mindestens einem Fettsäureglykolester der allgemeinen Formel 1 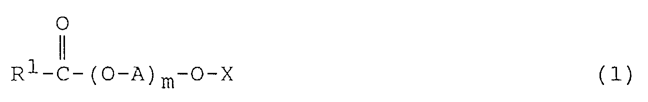 worin R¹ einen Alkylrest mit 12 bis 22 C-Atomen, A eine Gruppe der Formel -C₂H₄- oder -C₃H₆-, X ein Wasserstoffatom oder eine Gruppe der Formel 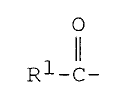 und m eine Zahl von 1 bis 10 bedeuten,
b) 5 bis 15 Gew.-% von mindestens einem Betain der allgemeinen Formel 2 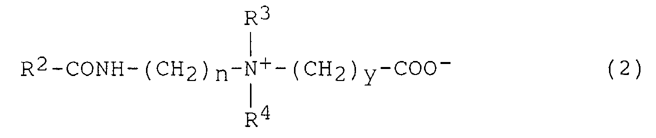 worin
R² einen Alkylrest mit 8 bis 22 C-Atomen,
R³ und R⁴ je einen Alkylrest mit 1 bis 3 C-Atomen,
n 2, 3 oder 4 und y 1, 2 oder 3 bedeuten,
c) 5 bis 18 Gew.-% von mindestens einem
Fettalkoholalkoxylat der allgemeinen Formel 3
R⁵-(O-B)_{z}-OH (3)
worin
R⁵ einen Alkylrest mit 8 bis 22 C-Atomen,
B eine Gruppe der Formel -C₂H₄- oder -C₃H₆- und
z eine Zahl von 1 bis 15 bedeuten, und
d) Wasser und geringe Mengen von Zusätzen, beispielsweise Konservierungsmittel, in der auf 100 Gew.-% fehlenden Menge,
dadurch gekennzeichnet, daß man
(1) die Komponenten b), c) und d) auf eine Temperatur von 70 bis 90 °C erhitzt zur Erreichung einer Lösung aus diesen Komponenten und getrennt davon die Komponente a) auf eine Temperatur von 5 bis 20 °C oberhalb des Schmelzpunktes erhitzt zur Erreichung einer homogenen Schmelze dieser Komponente,
(2) die homogen aufgeschmolzene Komponente a) unter Rühren zur Lösung aus den Komponenten b), c) und d) hinzufügt und die erhaltene Dispersion aus den Komponenten a) bis d) auf eine Temperatur von 70 bis 90 °C bringt, worauf man die Dispersion unter Weiterrühren auf eine Temperatur von 30 bis 40 °C abkühlen läßt, wobei die Komponente a) auskristallisiert, und daß man
(3) die im Schritt (2) erhaltene Dispersion mit auskristallisierter Komponente a) unter Weiterrühren und Anwendung von Kälte bis auf Raumtemperatur abkühlt, wobei die angestrebte Perlglanzdispersion erhalten wird, welche einen pH-Wert von 3 bis 8 aufweist.

## Claims

1. A process for the preparation of a free-flowing aqueous pearlescent dispersion comprising
a) from 15 to 35 % by weight of at least one fatty acid glycol ester of the formula 1 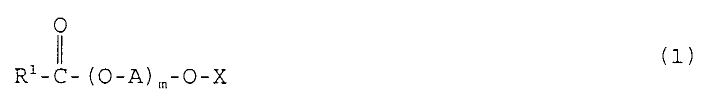 in which R¹ is an alkyl radical having 12 to 22 carbon atoms, A is a group of the formula -C₂H₄- or -C₃H₆-, X is a hydrogen atom or a group of the formula 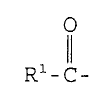 and m is a number from 1 to 10,
b) from 5 to 15 % by weight of at least one betaine of the formula 2 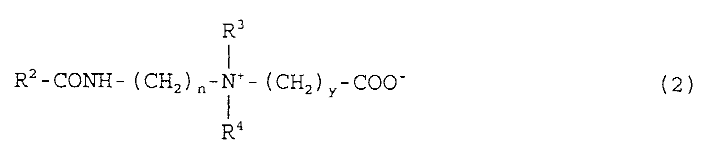 in which R² is an alkyl radical having 8 to 22 carbon atoms, R³ and R⁴ are each an alkyl radical having 1 to 3 carbon atoms, n is 2, 3 or 4, and y is 1, 2 or 3,
c) from 5 to 18 % by weight of at least one fatty alcohol alkoxylate of the formula 3
R⁵-(O-B)_{z}-OH (3)
in which R⁵ is an alkyl radical having 8 to 22 carbon atoms, B is a group of the formula -C₂H₄- or -C₃H₆-, and z is a number from 1 to 15, and
d) water and small amounts of additives, for example preservatives, in the remaining amount to 100 % by weight,
which comprises
(1) heating components b), c) and d) to a temperature of from 70 to 90°C in order to dissolve these components, and, separately therefrom, heating component a) to a temperature of from 5 to 20°C above the melting point in order to achieve a homogeneous melt of this component,
(2) adding the homogeneously melted component a) to the solution of components b), c) and d) with stirring, warming the resultant dispersion of components a) to d) to a temperature of from 70 to 90°C, and then allowing the dispersion to cool to a temperature of from 30 to 40°C with further stirring, during which component a) crystallizes out, and
(3) cooling the dispersion obtained in step (2) containing crystallized-out component a) to room temperature with further stirring and using cooling means, giving the desired pearlescent dispersion, which has a pH of from 3 to 8.

## Revendications

1. Procédé de préparation de dispersions aqueuses, à brillant nacré, aptes à l'écoulement, constituées
a) de 15 à 35 % en poids d'au moins un ester glycolique d'acide gras de formule générale 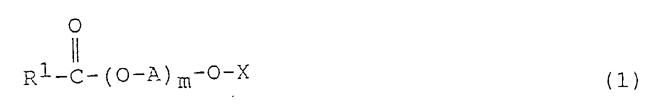 où R¹ représente un reste alkyle avec 12 à 22 atomes de carbone, A un groupe de formule -C₂H₄- ou -C₃H₆, X un atome d'hydrogène ou un groupe de formule 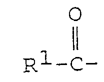 et m est un entier de 1 à 10,
b) de 5 à 15 % en poids d'au moins une bétaïne de formule générale 2 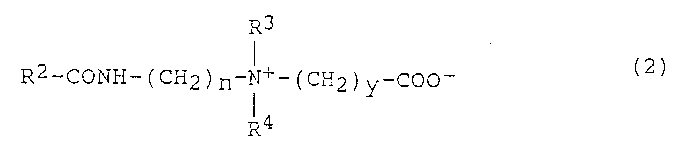 dans laquelle R² représente un reste alkyle avec 8 à 22 atomes de carbone, R³ et R⁴, chacun un reste alkyle avec 1 à 3 atomes de carbone, n vaut 2, 3 ou 4 et y vaut 1, 2 ou 3,
c) de 5 à 18 % en poids d'au moins un alcoxylate d'alcool gras de formule générale 3
R⁵-(O-B)_{z}-OH (3)
où R⁵ représente un reste alkyle avec 8 à 22 atomes de carbone, B un groupe de formule -C₂H₄- ou -C₃H₆ et z un nombre de 1 à 15, et
d) d'eau et de faibles quantités d'additifs, par exemple d'agents conservateurs, dans une quantité nécessaire pour 100 % en poids,
caractérisé en ce que
(1) l'on chauffe les composants b), c) et d) à une température de 70 à 90 °C pour obtenir une solution de ces composants et, dans une opération séparée, on chauffe le composant a) à une température de 5 à 20 °C supérieure au point de fusion pour obtenir une matière fondue homogène de ce composant,
(2) on ajoute le composant a) fondu homogène, en agitant, à la solution des composants b), c) et d) et l'on chauffe la dispersion obtenue des composants a) à d) à une température de 70 à 90 °C, en laissant refroidir la dispersion sous agitation à une température de 30 à 40 °C, le composant a) se séparant par cristallisation et en ce que
(3) l'on refroidit jusqu'à la température ambiante la dispersion obtenue dans l'étape (2) avec le composant a) cristallisé, en poursuivant l'agitation et en appliquant du froid, en obtenant la dispersion à brillant nacré voulue, qui présente une valeur de pH de 3 à 8.
